**Europäisches Patentamt**

(19) **European Patent Office**

**Office européen des brevets**

(11) Publication number: **0 183 717**

**B1**

(12) # EUROPEAN PATENT SPECIFICATION

(45) Date of publication of patent specification: **23.11.89**

(51) Int. Cl.⁴: **G 01 N 1/30, G 01 N 33/52, G 01 N 33/48, C 12 Q 1/04**

(21) Application number: **85902035.6**

(22) Date of filing: **02.05.85**

(86) International application number:
**PCT/EP85/00192**

(87) International publication number:
**WO 85/05446 05.12.85 Gazette 85/26**

(54) **SINGLE DYE REPLACEMENT FOR ROMANOWSKY PLURAL DYE VARIABLE COMPOSITIONS IN HUMAN BIOPSY SPECIMEN CONSTITUENT IDENTIFICATIONS.**

(30) Priority: **15.05.84 US 610381**

(43) Date of publication of application:
**11.06.86 Bulletin 86/24**

(45) Publication of the grant of the patent:
**23.11.89 Bulletin 89/47**

(84) Designated Contracting States:
**BE CH DE FR GB IT LI NL**

(56) References cited:
**FR-A-2 036 517**
**GB-A-2 074 749**
**GB-A-2 095 402**
**GB-A-2 116 712**

(73) Proprietor: **Kass, Lawrence**
**1939 Ridge Road**
**Hinckley Ohio 44233 (US)**

(72) Inventor: **Kass, Lawrence**
**1939 Ridge Road**
**Hinckley Ohio 44233 (US)**

(74) Representative: **Kador & Partner**
**Corneliusstrasse 15**
**D-8000 München 5 (DE)**

Courier Press, Leamington Spa, England.

## Description

Background of the Invention
Field of the Invention

This invention provides a single substantially pure class of dyestuff for use in cytological preparation of fixed biopsy specimens of human blood, bone marrow, blood smears, tissue specimens, etc. The single dyes of this invention substantially replace the prior art Romanowsky and Malachowski type mixed multiple dyes which are of variable composition and lacking in product shelf life constancy with age. Under standardized control conditions, before both and after use in preparing fixed cytological stained specimens for further diagnosis and study, the stain compositions have excellent color stability and are remarkably clear with respect to cellular detail and brilliance of cell structures such as granules.

It is submitted that this single class of aqueous panoptic dye will make obsolete and will supplant the later modifications of the above Romanowsky Derivative dye stains contributed by Jenner, Leishman, Wright, Giemsa and MacNeal. The latter Panoptic mixed dyes today remain the cornerstone of morphological hematology and are in universal use for identification of human components found in fixed human blood, bone marrow and tissue biopsy specimens.

Description of the Prior Art

Ehrlich in 1970 introduced the use of dyes in admixture to effect enhanced cell differentiation in human biopsy specimens, particularly blood cells. Ehrlich's dyes were later superseded in time by dye mixtures here broadly identified as Romanowsky Derived prior art dyes which were improved over time by progressive modifications embracing mixtures such as methylene blue, modified methylene blues, eosin, azures and methylene violet which admixtures have been generally classed as panoptic stains because of the wide range and broad spectra of hues and chroma produced when they are brought in contact with fixed biological specimen.

Starting in 1981, Romanowsky and Malachowski developed initial mixtures of polychromed methylene blue, azures and methylene violet. A chronology of contributors included such names as Unna (1891), Nocht (1898), Jenner (1899), Leishman (1901), Wright (1902), May-Grunwald and Giemsa (1902), MacNeal (1906), Lillie (1943) and others later all made contributions to what we here condense in a present generic state of the art label as Romanowsky Derived panoptic dye mixtures.

Composition developed by some of the foregoing talented contributors to the "state of the art" are indicated generally in the following Table I.

TABLE I
COMPOSITION OF COMMON PANOPTIC STAINS IN PRESENT USE

| | Eosin | Methylene Blue | polychromed Methylene Blue | Azure(s) | Methylene Violet |
|---|---|---|---|---|---|
| Romanowsky (1981) | | | X | X | X |
| Malachowski (1891) | | | X | X | X |
| Jenner (1899) | X | X | | | |
| Leishman (1901) | X | | X | | |
| Wright (1902) | X | | X | | |
| Giemsa (1902—04) | X | X | | | |
| MacNeal (1906) | X | X | | X | X |

The present state of the art discloses, so far as known, no single substantially pure commercially available dyestuff capable of panoptically and metachromatically staining a fixed biopsy specimen

2

containing human blood from venepuncture, bone marrow or tissue, for example, which permits differentiation and identification of individual leukocytes, sub-populations of lymphocytes, megakaryocytes, parasites carried in the blood, etc., of interest of diagnostic medical studies carried out over an extended time frame of study.

A single substantially pure dye permitting review of the slides and actual specimens prepared over such extended time frame, which dye does not substantially deteriorate in hue, value and chroma and remains usefully comparative, the old with the new, provides an advance in the art of considerable value to the advancement of cytological studies over an extended time frame.

It is recognized that the degree of purity and stability of the prior art dyes presently employed for the study of human biopsy specimens as represented by the Romanowsky Derived dye admixtures burdens the medical researcher with problems of reproducible examination and comparisons in sequential studies. More stable and permanent preservation of dye stained specimens of a fixed nature is a desired objective in the relevant art. The single substantially pure dyes of this disclosure provides a substantial advance in the field of differentiation and identification of leukocytes and other cell components having a locus in and from human biopsy specimens. HPLC analysis shows no substantial admixture of dyes present in the compound.

The substantially pure dye of this invention contains the chemical compound identified by its known structure. The dye is also designated and identified in the Color Index as basic blue 41 and No. 11154. This dye is also a panoptic dye and is further identified as having the somewhat unusual property of metachromasia.

The prior art dyes of the Romanowsky Derived qualities are not substantially chemically pure. They are known to contain substantial portions of many different dyes including, for example, methylene blue, polychromed methylene blue, azure A, azure B, azure C, thionin, methylene violet and azure B eosinate, etc.

The substantially pure dye of this invention is understood to contain primarily the structural compound of chemical formula associated with the color name basic blue 41, Color Index No. 11154. Reports from a private laboratory indicate that under HLPC or High Pressure Liquid Chromatography, the commerically available dye is unusually pure. It is a dye of the panoptic dye class. Metachromatic properties have been observed in the study of identifying color differences as set out in Table II where, initially each of the 17 different blood cell components have been differentiated and identified by color differences as will be noted.

Sub-populations of lymphocytes are also embraced.

Summary of the Invention

As introduced above, the present state of the art relies upon a variety of dyes used in combinations and permutations for identification through use of fixed blood cells, blood smears, bone marrow and hematopoietic tissue for diagnostic purpose.

This invention discloses a bright blue operative dye, substantially pure and listed in the Color Index as basic blue 41, Color Index No. 11154. It requires no chemical treatment or admixture with any other dyestuffs for the purposes of supplanting the earlier prior art dyestuffs. These prior art admixtures are reaction products and blends of a plurality of dyestuffs as set out above for general purposes.

Basic blue 41 (Color Index No. 11154) is in a class by itself. By comparison with basic blue 54 (Color Index No. 11052), basic blue 65 (Color Index No. 11076), basic blue 66 (Color Index No. 11075) and basic blue 67 (Color Index 11185), all of which are basic organic quaternary cationic azo dyes of greater complexity in substituent groups and higher molecular weight are essentially inoperative for the wide variety of end purposes as hereinafter set out.

In the best method presently known for reduction to practice of the present invention, films or smears of peripheral venous blood, including buffy coat preparation as appropriate in cases of pancytopenia, subleukemic leukemia, acute leukemia, etc.; or bone marrow particles obtained by sternal or iliac puncture; or blood smears in parasitic studies are produced between cleaned glass coverslips in the case of film preparations or on cleaned glass slides in the selection of smear preparations.

The coverslips or slides were air dried, fixed for two minutes in a 1% solution of Basic blue 41 in absolute methanol. Without washing, a previously prepared 1% aqueous solution of the test dye buffered to a pH of 7.6 with tris maleate buffer concentrate (200 mmole/1) which had been filtered prior to use was added to stain the fixed specimens for two or three minutes. The so prepared specimens were thereafter washed with distilled water at ambient temperature until bleeding of the dye from the prepared slides could no longer be detected. The washed slides or coverslips were mounted, face down on cleaned glass slides with a conventional mounting media such as Permount. As the work supporting this disclosure was carried forward under white light microscopy, it is anticipated that variations in the physical equipment may be carried forward by known and developing advanced high technology automatic differential analyzers which includes other means than noted here as illustrative but not limiting.

One of the principal advances in the use of the novel cationic dyestuffs of this invention is, however, the preparation of films and smears which may be preserved and reexamined for research which may take place over a period embracing many decades. Subjecting slides to a variety of tests to simulate age duplication indicates color stability and preservation of specimens prepared as indicated above, equal to or better than any prior art dyestuff presently known for the purposes herein. Novel dyes disclosed in US—A—4,400,370 and GB—A—2,116,712 all are of vital use in *non-fixed* specimens but are *not* known to retain the high degree of discrimination of blood cell components over long periods of storage of the vitally dyed biopsy specimens prepared in films of smears on glass.

A principal advantage of the bright basic blue dye of this invention is the long storage potential for records which can be periodically consulted over extended periods of time.

On the shelf, stability of a 1% distilled water solution of the basic blue dye 41 appears to be excellent. The dry dye in powder form appears to have a indefinitely long storage life.

Upon direct comparison of the dyes disclosed herein with the conventional panoptic staining dye mixtures developed between about 1890 and the present in the staining of human blood component cells, the following advantages have been observed:

1. A more rapid dye staining occurs, eliminating several minutes of staining time over the conventional panoptic stains of the prior art.

2. Only one substantially pure single lot of dyestuff is required rather than several lots of qualitatively different dyestuffs. As one might expect, there is better initial quality control with but a single stable dyestuff. Present panoptic dyes known are mixtures of a plurality of interblended and inter-reacted dyes whose proportions are inherently subject to variations in control and performance.

3. Interchemical reactions, as in the case of polychroming methylene blue, are not essential to achieve the wide range of colors observed on staining of specimens.

4. Storage life of the single basic dyestuff, both in the dry from, which is substantially of unlimited duration, and in the liquid aqueous form for many years are characteristic of the disclosed dyes.

5. No fading of the panoptic colors upon record storage of stained slides has been observed as compared with most known panoptic stained slides of the prior art.

6. Compared to the conventional Romanowsky-type staining mixtures, staining of the cells with basic blue 41 gives clearer details of nuclear chromatin and more brilliant coloration of granules.

7. Identification of established populations of lymphocytes including T-cells and B-cells, and related subpopulations of lymphocytes including: $T_{(s)}$, [T-suppressor cells]; $T_{(h)}$, T-helper cells and NK cells [Natural Killer cells] all of which are known to be important modulators or modifiers of the immune response in normal individuals can now be identified without delay.

8. Identification, differentiation and enumeration of the populations and sub-populations of lymphocytes including T-cells and B-cells which are a major lymphocyte population in man are basic to the advance of studies relating to the immune system and the various diseases associated as in lymphomas, lymphoid leukemias and immuno-deficiency states.

B-cells represent from about 10% to 20% of the peripheral blood lymphocytes and T-cells, which are indicators of the cellular immune responses, such as: delayed hypersensitivity, graft and organ transplant rejection, constitute about 70% to 80% of the total.

Sub-populations of lymphocytes known as $T_{(h)}$ or T-helper cells, $T_{(s)}$ or T-suppressor cells serve to regulate antibody production and are effectors of T-cell function. There are another recognized class of lymphocytes cell population which are not classed with these T-cells or B-cells and known as "null" cells. These may include "NK cells" or Natural killer cells which latter NK cells are also identified by the use of basic blue 41.

Enumeration of T-lymphocytes has diagnostic significance in certain chronic disorders where reduced numbers are found. Monitoring the numbers of T-suppressor and T-helper subsets has advanced understanding and treatment of diseases affecting the immune system. Changes in ratios of T-helper or T-suppressor cells has been assigned importance in allergy characterization. Monitoring relative changes in these numbers is of value in providing indicia of a possible rejection crisis in organ surgery transplant.

Chemotherapy patients have been monitored to maintain an immuno-competent state in guiding improvements in the prognosis of cancer patients. Decreasing β-cell and T-cell lymphocytic function in immuno-deficiency diseased patients may indicate bone marrow transplantation complications from clinical data studies of the immune system.

Heretofore, lymphocyte identification, particularly of the foregoing sub-populations were based upon their cell surface properties through use of specific monoclonal antibodies synthesized using novel techniques for antibody production and developments in genetic engineering.

Chronic lymphocytic leukemia was often characterized by a monoclonal proliferation of β-cells. More recently, a T-helper variant, a T-suppressor variant and a NK cell variant have been observed in which the leukemic lymphocytes share surface antigenic properties found in the corresponding normal lymphocyte sub-type.

Observed changes in the ratios of T-helper cells to T-suppressor cells have been frequently found in AIDS (Acquired Immune Deficiency Syndrome). NK cells are postulated to constitute the body's first line of defense against carcinogenic influences.

To date, however, there has not heretofore been an established or reproducible method of identification of differences between B-cells, T-suppressor cells, T-helper cells and Natural Killer or NK cells with any known panoptic stain.

No method is presently known for single stain of megakaryocytes to identify, distinguish or enumerate these cells from among all other marrow cells in a biopsy section. Staining of parasitic cells present in the blood are also accurately delineated.

9. Parasites from individuals known to be the victims of parasitic attack, as in tropical fevers due to Plasmodium Falciparum and Trypanosomiasis can be identified by their specific color and pattern, as with some of the prior art Romanowsky Derived dyes. Sharper contrasts have been noted with the pure dyes making clear diagnosis accurately. Basic blue 41 produces substantially the same coloration of the parasites, but the detail is obtained quicker with greater clarity of results.

TABLE II

| | CELL | NUCLEUS | CYTOPLASM | GRANULES |
|---|---|---|---|---|
| 1. | Erythrocytes | — | pale green | |
| 2. | Platelets | — | lavender | red, purple |
| 3. | Neutrophils | red, brown | cream color | red, purple |
| 4. | Lymphocytes | " | pale blue | rare red |
| 5. | Monocytes | " | lavender | red, pink |
| 6. | Eosinophils | " | colorless | bright green |
| 7. | Basophils | " | colorless | red-purple |
| 8. | Megakaryocytes | " | purple | red, crimson |
| 9. | Plasma cells | " | blue | — |
| 10. | Macrophage | " | gray | yellow, brown, green |
| 11. | Proerythroblasts | " | deep blue | — |
| 12. | Normoblasts | " | blue, gray-green | |
| 13. | Promyelocytes | " | blue | orange, red |
| 14. | Neutrophilic Myelocytes | " | yellow | orange, red |
| 15. | Neutrophilic Metamyelocytes | " | yellow | orange, brown |
| 16. | Band | " | cream color | orange, brown |
| 17. | Mast cells | " | colorless | red-purple |

In the erythroblast series, the range in color is from blue in the earliest erythroblast cytoplasm to gray-green in the cytoplasm of later normoblasts reflects increasing hemoglobinization of the cell (green color).

Maturation of granulocytic cells in the neutrophilic series, from promyelocyte to band, is reflected by difference in size, cytoplasmic volume, nuclear configuration, and number and color of granules (primary granules stain orange and red, whereas secondary granules stain brown).

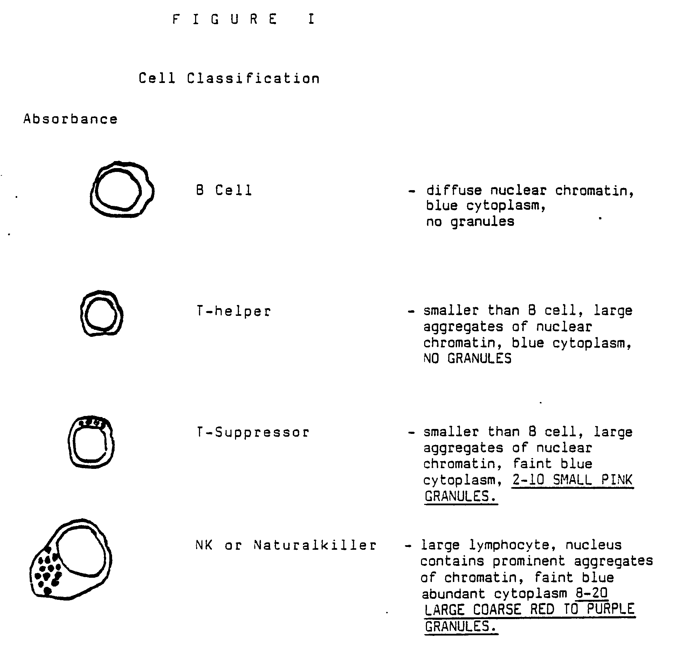

FIGURE 1 provides cell classification data relative to the sub-populations of lymphocytes and the means of their differentiation and identification basic to enumeration to establish specific detail.

While this application came into use by and through optical microscope study and through individual laboratory research, it is well-known in the medical diagnostics arts that optical microscopes have been and are supplanted by many technical advances wherein laser techniques and computer-type instrumentation have been advanced to supersede and advance identification, differentiation and enumeration of the normally invisible by advanced instrumentation. Thus, the term and word microscopic as used herein is intended broadly as generic to the univers of the infinitely small.

The nature of the microscopic biological components present in the human biopsy specimens to which the disclosed method is not fully explored. The use of the invention disclosed herein includes microscopic studies of human biopsy specimens which have been fixed and dyed with basic blue 41 and have been established by studies of bone marrow, hematopoietic or blood forming tissues, blood, blood fractions, blood smears, etc. These studies have made possible identification of the lymphocytes, sub-populations of lymphocytes, tropical disease parasites and inclusion of a wide variety of classification of elements present therein to permit further diagnosis and study of the diseases which attack living organisms and particularly man.

Identification, differentiation and enumeration of this individual world of the microscopically small is basic to the advances of studies relating to the immune system and the various diseases associated therewith as in lymphomas and lymphoid leukemias and immuno-deficiency states. T-cells and B-cells are the major lymphocyte sub-populations in man. B-cells represent from about 10% to 70% of the peripheral blood lymphocytes. T-cells are mediators of cellular immune responses, such as delayed hypersensitivity, graft and organ transplant rejection constitute 70% to 80% of the total. Certain other sub-populations including T-helper ($T_h$) and T-suppressor ($T_s$) cells regulate antibody production and effector T-cell function.

In certain chronic disorders patients may be found generally to have reduced numbers of T-lymphocytes. More recently it has been established advantageous to monitor subset numbers in analysis of $T_{(s)}$ and $T_{(h)}$ subsets of T-cell numbers as an aid to understanding and treatment of diseases affecting the auto immune system. Allergies may be characterized by changes in ratio of T-helper and T-suppressor subset numbers. Monitoring changes in these numbers may provide indicia of approach of a rejection arising in organ transplant surgery. Chemotherapy patients have been monitored as an aid to the physician in maintaining an immuno-competent state in guiding improvements in the prognosis of cancer patients.

Severe combined immuno-deficiency diseased patients suffering from decreasing B-cell and T-cell lymphocytic function may be aided by bone marrow transplantation through such clinical studies of the immune system.

Present studies performed in research laboratories relating to immunological research presently rely upon immunologic markers to identify specific lymphocyte sub-populations. T and B-cells were the first lymphocyte sub-populations to be so identified. The state of the art has now reached the point where T and B cell assays are a part of the routine in cytochemical examination, presently through use of the reaction of monoclonal antibodies, and earlier by use of the reaction for acid phosphatase.

Over the past several decades, it has become apparent that lymphocytes, once thought to be a single class of cells, are actually a heterogeneous collection of multiple sub-populations. Early studies established that there were at least two major types of lymphocytes in blood and in lymph nodes, namely T and B-cells. T-cells are believed to be involved in cell-mediation immunity, and constitute the largest number of lymphocyte in normal peripheral blood. B-cells are involved in humoral immunity and in the synthesis of antibody, and constitute a small proportion of the total number of lymphocytes in normal blood. At first, T and B-cells were distinguished from one another on the basis of their ability to form rosettes with sheep erythrocytes. B-cells formed rosettes with sheep red blood cells treated with complement. T-cells formed rosettes with untreated sheep red blood cells. In lymph nodes, B-cells are found predominantly in the germinal center of lymphoid follicles. T-cells are the predominant type of lymphocyte in the paracortical regions.

Shortly after the separation of lymphocytes into T and B-cell types it became apparent that some lymphocytes were neither T or B-cells and accordingly were named null cells. In an effort to more precisely characterize the properties of T and B-cells, various enzymatic and cytochemical techniques were applied to lymphocyte suspensions. Using this reaction for acid phosphatase, it was found that T-cells contained unipolar localization of the reaction product, whereas B-cells contained little, if any, activity of acid phosphatase. Similarly, nonspecific esterase activity is localized in a unipolar distribution of reaction product in T-cells, but in B-cells nonspecific esterase activity is diffuse or absent.

Another milestone in the detection of lymphocyte sub-populations occurred approximately a decade ago. Specific monoclonal antibodies against cell surface antigens were synthesized using newly developed immunologic techniques for antibody production. Soon, a wide variety of specific cell surface antibodies were developed for lymphocytes, and applied to the problem of lymphocyte identification. With this new technology, multiple sub-populations of lymphocytes were detected, based upon their cell surface properties. These sub-populations included B-cell, T-cell, T-helper cell, T-suppressor cell, and natural killer (NK) cell.

Concurrent with these developments, greater understanding of the role of the lymphocyte sub-populations in health and disease was taking place. Chronic lymphocytic leukemia was characterized as a monoclonal proliferation of B-cells, in most instances. More recently, other variants of chronic lymphocytic leukemia and of lymphocytic lymphoma have been described. In the case of chronic lymphocytic leukemia, these include a T-helper variant, T-suppresor variant, and natural killer variant, in which the leukemic lymphocytes share surface antigenic properties found in the corresponding normal lymphocyte sub-type.

Additional studies indicated that in normal individuals, T-helper and T-suppressor cells acted as modulators or modifier of the immune response. In systemic lupus erythematosis and in rheumatoid arthritis elevated levels of T-helper cells were found. The infectious mononucleosis, the atypical lymphocyte was characterized as T-suppresor cell. Within the past several years, changes in the ratio of T-helper cell to T-suppressor cell in the peripheral blood have been described as a frequent finding in patients with the Acquired Immune Deficiency Syndrome (AIDS). Currently, the finding of elevated levels of T-suppressor cells and either normal or reduced numbers of T-helper cells is one of the few diagnostic tests available for detection of patients with AIDS and individuals at risk for development of AIDS.

6

Natural killer cells (NK) have been subjects of active interest in many laboratories around the world for the past several years. Natural killer cells correspond to the large granular lymphocyte seen with conventional panoptic stains like Wright's or Giemsa's stain, and the granules are believed to be lysosomes since they contain phosphatase. Although the role of natural killer cells is not fully understood at the present time, these cells are believed to constitute the body's first line of defense against malignancy. Natural killer cells are able to directly kill a foreign or invading malignant cell without the mediation of complement. In parallel with developments in immunology, development of instrumentation, such as the fluorescence activated cell sorter, has also progressed rapidly and provides a powerful analytical tool for separation of the lymphocyte sub-populations in a highly purified form.

Although natural killer cells have been equated with the large granular lymphocytes as visualized with conventional panoptic stains, as yet there has not been a description or reproducible differences between B-cells, T-suppressor cells, T-helper cells, and natural killer cells with any panoptic stain. At present, differences between the lymphocyte sub-populations are detectable only with the use of monoclonal antibodies using tedious and expensive techniques, or with the less highly specific methods such as sheep red cell rosetting.

The following example illustrates the practice of the foregoing invention and the utility thereby:

## Example 1

A male patient, age 40, developed weakness and fever. Physical examination revealed an enlarged spleen. Laboratory studies further showed hemoglobin 9 grams %, white blood cell count of $278,000/mm^3$ and a platelet count of $684,000/mm^3$. A series of films or smears of peripheral venous blood and bone marrow particles were made between cleaned glass coverslips for film preparations and on cleaned glass slides for smears. After air drying, the specimens were fixed for five minutes in absolute methanol at room temperature (25°C) with a 1% solution of basic blue 41 for five minutes.

The so prepared fixed coverslips and slides were washed with distilled water until no further bleeding of the dye color from the slide was observed. The water-washed slides and coverslips were again air dried. The coverslips were mounted face down on cleaned glass slides with conventional (Permount) mounting media. These were then subject to white light illumination and microscopic examination.

The permanent slides so prepared exhibited a striking increase over normal in mature and immature granulocytic cells and increased number of basophils. Diagnosis of chronic granulocytic leukemia was confirmed by the presence of the Philadelphia chromosome upon further cytogenetic analysis of the bone marrow.

## Example 2

A 78 year old female noticed sores in her mouth and bleeding gums. Routine blood count determined hemoglobin of 8 grams %, white blood cell count $3500/mm^3$ with 65% monocytes, platelet count $4000/mm^3$.

Peripheral blood and bone marrow films stained with a 1% solution of basic blue 41 in distilled water and were further prepared as in Example 1. Increased numbers of monocytes possessing red-brown staining of nuclear chromatin, nuclear lobulations and abundant cytoplasm that stained pale lavender containing variable numbers of red and pink granular structures were observed. A dublicate set of slides separately prepared from the patient's blood using Wright's stain (prior art) revealed monocytes stained similarly to the above blood stained slides in hue and chroma using basic blue 41. The above dual analyses were confirmatory consistent with a finding of acute Myelomocytic Leukemia.

## Example 3

An 18 year old male college student developed fever, enlarged cervical lymph nodes and pharyngitis. Physical examination revealed a tender, enlarged spleen. Laboratory studies showed hemoglobin 13 grams %, white blood cells $25,000/m^3$ and platelets of $175,000/mm^3$.

Peripheral blood smears prepared as in Example 1 using an aqueous 1% solution of basic blue 41 determined 80% atypical lymphocytes. These contained a red-brown stained nucleus with block-like aggregates of nuclear chromatin and abundant cytoplasm staining pale blue in some lymphocytes. Other lymphocytes were observed staining a brighter blue with radial striation. A similar slide made at the same time using Wright's stain, a standard prior art panoptic staining mixture, produce atypical lymphocytes having substantially similar hue and chroma as had been first perceived using basic blue 41, confirming the factual basis for diagnosis. As confirmed by serological testing, the diagnosis was infectious mononucleosis.

## Example 4

An 49 year old male had no detectable abnormalities upon physical examination. On routine laboratory analysis he was found to have an elevated white blood cell count (white blood cell count $22,000/mm^3$), hemoglobin 14 gram %; and platelets $250,000/mm^3$.

Slides prepared as in Example 1 consisting of peripheral blood smear and bone marrow smear stained with basic blue 41, aqueous, were examined under white light microscopy. Found were approximately 80 to 90% mature lymphocytes identified by red-brown staining nuclei containing well defined aggregates of nuclear chromatin and pale blue agranular cytoplasm. As confirmed by a Wright's-stained slide made at the same time, the diagnosis was chronic lymphocytic leukemia.

Example 5

During physical examination a 56 year old woman reported fever and abdominal pain. The locus of the pain was in the right lower quadrant. Intestinal perforation was indicated by X-ray of the abdomen showing free air. Hemoglobin was 10 grams %, white blood cell count 38,000/mm$^3$ and platelet count 450,000/mm$^3$. A peripheral blood smear and a smear of bone marrow particles obtained by a biopsy were fixed as in Example 1 and stained with an 1% aqueous solution of basic blue 41.

Microscopic examination of the above prepared Permount slides established increased numbers of bands having cream colored cytoplasm and orange-brown granules. Neutrophils showed a segmented nucleus that stained red-brown, and cream colored cytoplasm containing red and brown granules.

Except for lack of nuclear segmentation, the bands were similarly responsive to the stain indicia as were the neutrophils. Metamyelocytes had kidney-bean shaped nucleus, stained red-brown; a yellow cytoplasm containing orange and brown granules was noted. Myelocytes exhibited a round to oval red to brown stained nucleus and a yellow cytoplasm containing orange and brown granules. Promyelocytes had similar shape and color nucleus, but the cytoplasm stained blue with orange and red colored granules.

Similarly prepared comparative slides using Wright's stained biopsy specimens of the patient's blood and bone marrow upon comparison with the above demonstrated substantially the same hue and chroma as reported above. However, erythrocytes stained with basic blue 41 were pale green, while with Wright's stain were orange in color. This is indicative of the affinity of the hemoglobin of the erythrocytes for eosin found in Wright's plural dye containing panoptic staining mixtures. As confirmed by Wright-stained slides of blood and bone marrow made at the same time, the diagnosis of probable leukemoid reaction was mad. After removal of a perforated appendix, the blood abnormalities disappeared.

Example 6

Exacerbation of bronical asthma of a 32 year old female admitted her to the hospital. Frequent wheezing of the chest was observed. Laboratory studies report hemoglobin 13 grams %; white blood cell count 15,000/mm$^3$; platelet count 325,000/mm$^3$.

A peripheral blood smear stained with a solution (1%) basic blue 41 dye in distilled water established the presence of 30 to 40% eosinophils. Microscopic identification was characterized by a bi-lobed red-brown staining of the nucleus. The cytoplasm was filled with bright green stained oval to round granules. (Wright's stained granules of eosinophils ran in comparison were orange, again indicating affinity of the granules for the eosin component of the plural dye mixture of this panoptic stain.)

Example 7

A 80 year old man was admitted to the hospital complaining of profound weakness and fatigue. On physical examination he was pale and vital signs were normal. Laboratory data included homoglobin 9 grams %, white blood cell count 22,000/mm$^3$, and platelet count 650,000/mm$^3$. peripheral blood film stained with Wright's stain and a separate film stained with basic blue 41 both revealed increased numbers of platelets with many giant bizarre appearing platelets. Increased numbers of immature white blood cells (promyelocytes, myelocytes, metamyelocytes) and nucleated red blood cells were seen, along with tailed poikilocytes. Attempts at aspiration of bone marrow resulted in a "dry tap", but a bone marrow biopsy fixed in B5-formalin and sectioned in the usual way showed hypercellularity, increased fibrosis, and increase numbers of megakaryocytes using an H and E (hemotoxylin and eosin) stain. Using a 0.1% aqueous solution of basic blue 41 as a stain on a separate slide, magakaryocytes stained bright pink, whereas all other bone marrow cells stained pale blue. Using basic blue 41, megakaryocytes were easily and immediately visible as a result of their distinct color compared to other marrow cells, and their number could be readily quantitated. A diagnosis of agnogenic myeloid metaplasia with myelofibrosis was-made.

Example 8

A 55 year old woman was admitted on the hospital because of red spots on her lower legs, occuring over a period of 48 hours prior to admission. On physical examination, she was pale. The skin of both lower legs showed multiple petechiae. The tip of the spleen was palpable. Laboratory values included hemoglobin 12 gram %, white blood cell count 13,000/mm$^3$, and platelet count 3,000/m$^3$. Wright's stained, as well as separately basic blue 41 stained coverslips of peripheral blood, showed normal appearing white blood cells but striking decrease in the number of platelets. Bone marrow aspiration stained with either Wright's stain or basic blue 41 demonstrated increase numbers of young appearing megakaryocytes. On bone marrow biopsy fixed in B5-formalin and sectioned in the usual way, H and E stain showed increased numbers of megakaryocytes.

However, a separate section of bone marrow biopsy stained with basic blue 41 demonstrated striking numbers of megakaryocytes that stained bright pink, whereas all other bone marrow cells stained pale blue. A diagnosis of idiopathic thrombocytopenic purpura (ITP) was made.

## EP 0 183 717 B1

### Example 9

In another series of examples the aqueous blue stain solution comprising basic blue 41 was applied to a series of human bone marrow biopsies, just fixed in B5-formalin embedded in parrafin, sectioned on a microtome. Here basic blue 41 was established as a selective stain for megakaryocytes which cells stained a bright pink, while all other bone marrow cells stained blue. The test were performed as follows:

Parrafined sections of bone marrow, spleen and lymph nodes were conventionally prepared, brought to water after successive rehydration exposures through a series of concentrations from absolute alcohol dilutions.

Rehydrated sections were stained for 2 minutes with a 0.1% aqueous solution of basic blue 41 after washing in distilled water, the sections were immersed briefly in a series of alcohols of increasing alcohol concentration to dehydrate and then immersed in xylene.

A glass coverslip was applied over the tissue section with Permount.

Upon study under a light microscope of the several slides so prepared, megakaryocytes of varying maturational ages in both health and disease demonstrated bright pink staining of the cytoplasm. No other cell in the specimens stained this color. Other marrow cells stained blue. Sections known to contain other giant type cells including osteoclasts, Reed-Sternberg cells and Laughans giant cells, pink staining of the cytoplasm did not occur. Megakaryocytes, which stained bright pink, were identified, differentiated and enumerated easily.

### Example 10

Of interest was a series of tests where diastase was used to remove glycogen; ribonuclease to remove DNA, staining with basic blue 41 was not impaired. However, upon removal of acid mucopolysaccharide with hyaluronidase the pink color of the cytoplasm of the megakaryocytes failed to develop.

### Example 11

A 75 year old white male was admitted to the hospital with fever and chills. On physical examination, temperature was 39.7°C (103.5°F). He had enlarged lymph nodes and an enlarged spleen. Laboratory studies revealed hemoglobin 10.2 grams %, white blood cell count 8.500/mm$^3$, and platelet count 100,000/mm$^3$. Using Wright's stain, a differential count on a peripheral blood film showed approximately 85% lymphocytes and 15% neutrophils.

Using basic blue 41, the peripheral blood film revealed 85% lymphocytes and 15% neutrophils, and the lymphocytes were medium sized with diffuse nuclear chromatin and basophilic cytoplasm devoid of granularity. With a more tedious immunoperoxidase stain, the lymphocytes were confirmed as B-cells.

Diagnosis: Chronic Lymphocytic Leukemia.

### Example 12

A 32 year old black female was admitted to the hospital with a two week history of ankle adema, shortness of breath, and visual loss. On physical examination, vital signs were normal. An erythrematuous facial eruption along with an enlarged spleen was noted. Funduscopic examination disclosed multiple small white blood cell count 15,000/mm$^3$, and platelet count 65,000/mm$^3$. A differential cell count on a peripheral blood film stained with Wright's stain showed a normal differential with normal appearing lymphocytes. A differential cell count on the peripheral blood stained with basic blue 41 showed increased numbers of small lymphocytes, corresponding to T-cells. Also, the small lymphocytes having deep basophilic cytoplasm and no visible granules comprising approximately 70% of the total number of small lymphocytes. With immunoperoxidase staining, approximately 70 to 75% of the small lymphocytes were identifiable as T-helper cells. Other laboratory data included a positive ANA test and a positive LE cell test.

Diagnosis: Systemic Lupus Erythrematosis.

### Example 13

A 28 year old white male was admitted to the hospital with a one months history of cough, fever, lymphadenopathy, and weakness. On physical examination, he was pale, and vital signs were normal. Enlarged lymph nodes were found in the cervical, inguinal, and auxillary areas. Laboratory data included hemoglobin 10 gram %, white blood cell count 14,500/mm$^3$, and platelet count 125,000/mm$^3$. Wright's stained peripheral blood differential revealed normal numbers of lymphocytes, and no obvious differences between lymphocytes could be ascertained. On a peripheral blood film stained with basic blue 41, the majority of the lymphocytes were small, and of these small lymphocytes, approximately 60% had 6 to 10 tiny pink granules. Upon immunoperoxidase staining, these small lymphocytes corresponded to T-suppressor cells, creating an inversion of the normal T-helper/T-suppressor ratio. These laboratory and clinical finding were consistent with the diagnosis of Acquired Immune Deficiency Syndrome (AIDS). Subsequently, the patient expired with an opportunistic infection.

### Example 14

A 25 year old white male serviceman on leave to U.S.A. after a tour of duty in southeast Asia, was admitted with fever and chills of 3 days duration. Physical examination revealed a pale skin, BP 120/70, pulse 100, temperature 104°F. An enlarged spleen could be felt in the abdomen. Laboratory values included

9

hemoglobin 10 grams %, white blood cell count 22,000/mm³ with increased numbers of neutrophils and bands, platelet counts 425,000/mm³. Bilirubin was 3.2 mg %.

A thick smear of peripheral blood stained with Giemsa's stain revealed parasitic infestation of erythrocytes with the malarial parasite Plasmodium Falciparum. A comparative dublicated thick blood smear stained rapidly with basic blue 41 revealed unusual vived coloration of malarial parasites with colors substantially identical to those seen with the prior art Giemsa's stain. Malaria was diagnosed with accuracy and dispatch.

### Example 15

In another similar case to the foregoing, the parasite causing Trypanosomiasis stained red-purple in a blood smear and was readily identified with basic blue 41. Confirmation was made with use of a Romanowsky dye.

### Example 16

Using 125 cc of normal human blood from normally healthy volunteers, Ficoll-Hypaque gradients were used to isolate lymphocytes. The isolated lymphocytes were then treated with specific lymphocytic antigens, and separated into highly purified fractional populations by means of a commercial fluorescence activated cell sorter. B-cells were identified with antihuman total immunoglobuline F(ab)₂ fragment, OKT-4 for T-helper cells, OKT-8 for T-suppressor cells and Leu 7 for natural killer cells. After sorting, the lymphocytes were isolated to about a 95% purity according to their sub-populations. After the sorting step, the fractions were further concentrated with a cytocentrifuge. Each one of the so concentrated, purified lymphocyte sub-populations were deposited on separate clean glass slides, air dried and further fixed with a 1% solution of the basic blue 41 dye (C.I. 11154) in absolute methanol for 2 minutes. Without washing, a 1% aqueous solution of basic blue 41 bufferd with tris maleate to pH 7.6 was added, and stained for 2-3 minutes with the buffered dye. Wash with distilled water to remove all excess dye, air dry and Permount on a clean glass slide for conventional light microscopy.

B-lymphocytes stained with basic blue 41 were large lymphocytes with basophilic agranular cytoplasm. The nuclei contained diffuse-appearing chromatin with apparently smooth aggregates. Occasionally, one noted the nuclei of the B-cells to be eccentrically placed with the cell having some resemblence to plasma cells (See Fig. 1).

T-helper cells were smaller than the B-cells with large, darkly stained nucleus chromatin with prominent block-like aggregates of chromatin within the nucleus, the cytoplasm being free from granules and a deeply basophilic blue.

The T-suppressor cells were also small lymphocytes of the same general size of the T-helper cells with darkly stained nuclei containing prominent block-like aggregates of nuclear chromatin and scant less deeply blue cytoplasm. A prominent difference over the T-helper cells the T-suppressor cells (identified with the OKT-8 antibody contained 2 to 8 pink to red granules in the cytoplasm).

The NK cells (natural killer cells) were observed to be unusually large lymphocytes containing large round to oval nucleus containing both diffuse and block like aggregates of chromatin and a noticeably more voluminuous or abundant cytoplasm than the cells described above containing a generally increased number of from about 4 to 20 larger or coarser red to purple stained granules.

Attempts at aspiration of bone marrow resulted in a "dry tap", but a bone marrow biopsy fixed in B5-formalin and sectioned in the usual way showed hypercellularity, increased fibrosis, and incresed numbers of megakaryocytes using an H and E (hemotoxylin and eosin) stain.

Using a 0.1% aqueous solution of basic blue 41 stain on a separate slide, megakaryocytes were stained bright pink. All other bone marrow cells stained pale blue. Megakaryocytes were easily and immediately visible as a result of their distinct color compared to other marrow cells, and their number could be readily quantified.

## Claims

1. A method of differentiation and identification one from the other, among a plurality of cells present in a biopsy specimen including human hematopoietic blood forming tissue, blood smears, and bone marrow specimens which comprises fixing said biopsy specimen, staining said fixed specimen with a single quaternary azo dye soluble in pure water and absolute methanol known as basic blue 41 and further identified by Color Index No. 11154, thereby differentiating said plurality of the so stained cell entities present by the colors and patterns of colors characteristic of the each of individual stained cell components.

2. A composition comprising the reaction product of a plurality of human biological component cells from a biopsy specimen, illustratively a blood smear, a human blood specimen, and/or bone marrow, with a fixative and with a staining amount of a quaternary azo dye soluble in pure water and absolute methanol identified as basic blue 41 and identified by the Color Index No. 11154, said composition having utility in diagnostic examination and analysis in the treatment of disease.

# EP 0 183 717 B1

## Patentansprüche

1. Verfahren, um aus einer Vielzahl von in einer Biopsie-Probe vorhandenen Zellen eine von der anderen zu unterscheiden, einschließlich hämapoetisches Humanblut bildendes Gewebe, Blutausstriche und Knochenmarksproben, bei dem diese Biopsie-Probe fixiert wird, die fixierte Probe mit einem einzelnen quaternären Azofarbstoff gefärbt wird, der in reinem Wasser und absolutem Methanol löslich ist, als Basisch Blau 41 bekannt ist und darüberhinaus durch den Farbindex No. 11154 gekennzeichnet ist, wodurch die Vielzahl der so gefärbten vorhandenen Zelleinheiten durch den Kennwert der Farben und Farbmuster jeder einzelnen gefärbten Zellkomponente unterschieden werden.

2. Zusammensetzung, die das Reaktionsprodukt einer Vielzahl biologischer Humanzellkomponenten aus einer Biopsie-Probe, z.B. ein Blutausstrich, eine Humanblutprobe und/oder Knochenmark, mit einem Fixiermittel und mit einer färbenden Menge eines quaternären Azufarbstoffes umfaßt, der in reinem Wasser und absolutem Methanol löslich ist, als Basisch Blau 41 bezeichnet wird und durch den Farbindex No. 11154 identifiziert wird, wobei diese Zusammensetzung in der Diagnoseprüfung und bei der Analyse bei der Behandlung einer Krankheit Verwendung findet.

## Revendications

1. Méthode de différenciation et d'identification de l'un de l'autre parmi une pluralité de cellules présentes dans un spécimen de biopsie, à l'inclusion de tissu humain hématopoïétique favorisant la formation des éléments du sang, de prélèvements de sang et de prélèvements de moelle osseuse, comprenant la fixation du dit spécimen de biopsie, la coloration du dit spécimen fixé au moyen d'un colorant azoïque quartenaire unique soluble dans l'eau pure et le méthanol absolu, connu comme bleu basique 41 et en outre identifié par l'indice de couleur no. 11154, la dite pluralité des entités cellulaires présentes ainsi colorées étant différenciées à l'aide des couleurs et des structures de couleurs caractéristiques de chacun des composants cellulaires colorés individuels.

2. Une composition comprenant le produit de réaction d'une pluralité de cellules constitutives biologiques humaines d'un spécimen de biopsie, pour illustration un prélèvement de sang, un spécimen de sang humain et/ou de la moelle osseuse avec un fixatif et avec un colorant azoïque quartenaire en quantité suffisante pour la coloration, soluble dans l'eau pure et le méthanol absolu, identifié comme bleu basique 41 et identifié par l'indice de couleur no. 11154, la dite composition étant utile à l'examen et l'analyse diagnostique pour le traitement de maladies.

F I G U R E   I

Cell Classification

Absorbance

B Cell
- diffuse nuclear chromatin, blue cytoplasm, no granules

T-helper
- smaller than B cell, large aggregates of nuclear chromatin, blue cytoplasm, NO GRANULES

T-Suppressor
- smaller than B cell, large aggregates of nuclear chromatin, faint blue cytoplasm, 2-10 SMALL PINK GRANULES.

NK or Naturalkiller
- large lymphocyte, nucleus contains prominent aggregates of chromatin, faint blue abundant cytoplasm 8-20 LARGE COARSE RED TO PURPLE GRANULES.